(19) Europäisches Patentamt
European Patent Office
Office européen des brevets

(11) Veröffentlichungsnummer: **0 041 672**
B1

(12) **EUROPÄISCHE PATENTSCHRIFT**

(45) Veröffentlichungstag der Patentschrift: **28.09.83**

(51) Int. Cl.³: **C 07 C 47/54,** C 07 C 47/542, C 07 C 47/544, C 07 C 47/55, C 07 C 45/43 // C07C63/70, C07C63/04, C07C43/168

(21) Anmeldenummer: **81104166.4**

(22) Anmeldetag: **01.06.81**

(54) **Verfahren zur Herstellung von aromatischen Aldehyden.**

(30) Priorität: **10.06.80 DE 3021701**

(43) Veröffentlichungstag der Anmeldung: **16.12.81 Patentblatt 81/50**

(45) Bekanntmachung des Hinweises auf die Patenterteilung: **28.09.83 Patentblatt 83/39**

(84) Benannte Vertragsstaaten: **CH DE FR GB IT LI**

(56) Entgegenhaltungen: **DE-A-2 752 612**
**DE-B-1 618 157**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Im Geusfelde 35, D-5068 Odenthal (DE)**
Erfinder: **Wolters, Erich, Dr., Streffenweg 22, D-5162 Niederzier (DE)**

**EP 0 041 672 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von aromatischen Aldehyden

Die Erfindung betrifft ein Verfahren zur Herstellung von aromatischen Aldehyden durch Umsetzung von Benzalhalogeniden mit wäßriger Ameisensäure bei erhöhter Temperatur.

Es ist bekannt (DE-AS 1 618 157), Halogen- oder Halogennitrobenzaldehyde durch Verseifung von entsprechend substituierten Benzalhalogeniden mit mindestens stöchiometrischen Mengen Ameisensäure bei einer Temperatur zwischen 0 und 130° C in Gegenwart von Chloriden, Bromiden oder Jodiden der Metalle der II., VII., VIII. Nebengruppe, III. oder V. Hauptgruppe des periodischen Systems herzustellen.

Dieses Verfahren besitzt jedoch den Nachteil, daß sich die Aufarbeitung des Reaktionsgemisches durch die Anwesenheit der Metall-Halogenid-Katalysatoren, die ja von dem zu isolierenden Aldehyd abgetrennt werden müssen, technisch aufwendig gestaltet.

Außerdem können durch die Metall-Halogenid-Katalysatoren Nebenreaktionen, wie Friedel-Crafts-Reaktionen, hervorgerufen werden, wodurch die Ausbeute an Aldehyd vermindert wird.

Weiterhin ist aus J. Chem. Soc. 1926, 218, 220 bekannt, Chlorbenzalchlorid bzw. Fluorbenzotribromid und Fluorbenzalbromid mit der zweifachen Volumenmenge an 98%iger Ameisensäure bei Rückflußtemperatur zum entsprechenden Benzaldehyd und zur entsprechenden Benzoesäure zu verseifen. Nachteilig bei diesem Verfahren ist, daß große Mengen an hochkonzentrierter Ameisensäure verwendet werden, wodurch die Wirtschaftlichkeit des Verfahrens beeinträchtigt wird. Des weiteren wird auch in diesem Verfahren von der Anwesenheit von Phosphorpentachlorid und seinen Hydrolyse-Umwandlungsprodukten Phosphoroxichlorid und Orthophosphorsäure Gebrauch gemacht, die katalytisch wirksam sind.

In DE-OS 2 752 612 ist ein Verfahren zur Hydrolyse von Benzalchlorid zu Benzaldehyd mit überschüssiger wäßriger Salzsäure als Katalysator bei höherer Temperatur beschrieben. Hierbei muß die 6—40fache, bevorzugt 10—25fache Wassermenge, bezogen auf die stöchiometrisch notwendige Menge, im Reaktionsgemisch vorhanden sein, um nicht unwirtschaftlich lange Verseifungszeiten zu erfordern.

Es wurde nun ein Verfahren zur Herstellung von aromatischen Aldehyden der Formel

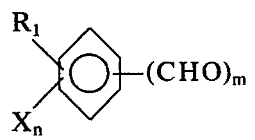

worin

R für Wassertoff, eine Carboxylgruppe, einen Alkylrest mit 1 bis 6 Kohlenstoffatomen, einen Cycloalkylrest mit 5 bis 9 Kohlenstoffatomen, einen gegebenenfalls mit Halogen ein- oder mehrfach substituierten Phenylrest oder einen gegebenenfalls mit Halogen ein- oder mehrfach substituierten Phenoxyrest steht,
X für Halogen steht,
l die Zahlen 0, 1, 2 bedeutet,
m die Zahlen 1, 2 bedeutet und
n die Zahlen 0, 1, 2, 3 bedeutet,

durch Umsetzung der entsprechenden Benzalhalogenide mit Ameisensäure bei erhöhter Temperatur gefunden, das dadurch gekennzeichnet ist, daß man die Umsetzung mit der 0,3- bis 7fachen stöchiometrischen Menge Ameisensäure ohne Verwendung eines Katalysators durchgeführt.

Als Benzalhalogenide können solche der allgemeinen Formel

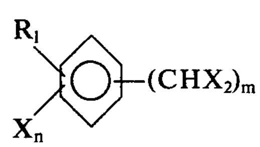

worin

R für Wasserstoff, eine Carboxylgruppe, einen Alkylrest mit 1 bis 6 Kohlenstoffatomen, einen Cycloalkylrest mit 5 bis 9 Kohlenstoffatomen, einen gegebenenfalls mit Halogen ein- oder mehrfach substituierten Phenylrest oder ein gegebenenfalls mit Halogen ein- oder mehrfach substituierter Phenoxyrest steht,
X für Halogen steht,
l die Zahlen 0, 1, 2 bedeutet,
m die Zahlen 1, 2 bedeutet und
n die Zahlen 0, 1, 2, 3 bedeutet,

in das erfindungsgemäße Verfahren eingesetzt werden.

Als Alkylreste mit 1 bis 6 Kohlenstoffatomen seien genannt: der Methyl-, Ethyl-, n-Propyl-, iso-Propyl-, n-Butyl-, iso-Butyl-, tert.-Butyl-, n-Amyl-, sec.-Amyl-, tert.-Amyl-Rest, bevorzugt der iso-Propyl-, tert.-Butyl-, tert.-Amyl-Rest;

als Cycloalkylreste mit 5 bis 9 Kohlenstoffatomen seien genannt: der Cyclopentyl-, α-Methylcyclopentyl-, α-Ethylcyclopentyl-, α-n-Propylcyclopentyl-, α-iso-Propylcyclopentyl-, Cyclohexyl-, α-Methylcyclohexyl-, α-Ethylcyclohexyl-, α-n-Propylcyclohexyl-, α-iso-Propylcyclohexyl-Rest, bevorzugt der Cyclopentyl- und α-Methylcyclopentylrest;

als gegebenenfalls mit Halogen, wie Fluor, Chlor, Brom, ein- oder mehrfach substituierte Phenylreste seien genannt:

der Fluorphenyl-, Chlorphenyl-, Dichlorphenyl-, Bromphenyl-, Phenylrest, bevorzugt der Fluorphenyl-, Chlorphenyl-, Dichlorphenylrest;

als gegebenenfalls mit Halogen, wie Fluor, Chlor, Brom, ein- oder mehrfach substituierte Phenoxyreste seien genannt:

der Fluorphenoxy-, Chlorphenoxy-, Dichlorphenoxy-, Bromphenoxy-, Phenoxyrest, bevorzugt der Fluorphenoxy-, Chlorphenoxy-, Dichlorphenoxy-, Phenoxyrest.

Als Halogene seien genannt: Fluor, Chlor, Brom und Jod, bevorzugt Fluor, Chlor und Brom, insbesondere Fluor und Chlor.

In das erfindungsgemäße Verfahren können folgende Benzalhalogenide eingesetzt werden:

4-Chlorbenzalchlorid, 2-Chlorbenzalchlorid, 4-Brombenzalbromid, Benzalchlorid, 4-Isopropylbenzalbromid, 4-tert.-Butylbenzalbromid, 4-tert.-Amylbenzalbromid, 4-Cyclopentylbenzalbromid, 4-α-Methylcyclopentylbenzalbromid, 4-[4′-Fluorphenyl-]benzalbromid, 4-[4′-Chlorphenyl-]benzalbromid, 4-[3′,5′-Dichlorphenyl-]benzalbromid, 3-[3′-Fluorphenoxy-]benzalbromid, 3-[3′-Chlorphenoxy-]benzalbromid, 3-[3′,5′-Dichlorphenoxy-]benzalbromid, 3-Phenoxybenzalbromid, 3-Phenoxybenzalchlorid, 3-Phenoxy-4-fluorbenzalchlorid, 3-Phenoxy-4-fluorbenzalbromid, 3-Brom-4-fluorbenzalchlorid, 3-Brom-4-fluorbenzalbromid, 3-Fluorbenzalchlorid, 4-Fluorbenzalbromid, bevorzugt: 4-tert.-Butylbenzalbromid, 4-Chlorbenzalchlorid, 2-Chlorbenzalchlorid, 3-Phenoxybenzalchlorid, 3-Phenoxybenzalbromid, 3-Phenoxy-4-fluorbenzalchlorid, 3-Phenoxy-4-fluorbenzalbromid, 3-Brom-4-fluorbenzalchlorid, 3-Brom-4-fluorbenzalbromid, 4-Fluorbenzalchlorid und 4-Fluorbenzalbromid.

Neben den reinen Benzalhalogeniden können auch Benzalhalogenide eingesetzt werden, die noch mit Trihalogenmethylbenzolen oder Monohalogenmethylbenzolen verunreinigt sind. Zum Beispiel können Benzalhalogenide eingesetzt werden, die noch etwa 1 bis 90%, bevorzugt 1 bis 40%, der entsprechenden Trihalogenmethylbenzole bzw. der entsprechenden Monohalogenmethylbenzole enthalten.

In diesen Fällen werden die Trihalogenmethylbenzole während der Reaktion in die entsprechenden Benzoesäuren überführt, während die Monomethylhalogenbenzole keine Veränderung erfahren.

Beispielsweise können als Ausgangsverbindungen Gemische von 4-tert.-Butylbenzalbromid, 4-Chlorbenzalchlorid, 2-Chlorbenzalchlorid, 3-Phenoxybenzalchlorid oder 3-Phenoxybenzalbromid, die noch ca. 1 bis 40% des entsprechenden Trihalogenmethylbenzols und/oder 1 bis 40% des entsprechenden Monohalogenmethylbenzols enthalten, in das erfindungsgemäße Verfahren eingesetzt werden.

Nach dem erfindungsgemäßen Verfahren wird die Ameisensäure bevorzugt als etwa 30- bis 95gew.-%ige, besonders bevorzugt als 85gew.-%ige, wäßrige Ameisensäure eingesetzt.

Die Ameisensäure wird im allgemeinen in der 0,1- bis 7fachen stöchiometrischen Menge, bevorzugt in der 0,3- bis 5fachen stöchiometrischen Menge, besonders bevorzugt in der 0,5- bis 2fachen stöchiometrischen Menge, in das erfindungsgemäße Verfahren eingesetzt.

Die Reaktionstemperaturen können in weiten Grenzen schwanken und betragen je nach eingesetztem Ausgangsprodukt 40 bis 150° C. Bevorzugt wird die Umsetzung bei Temperaturen von 70 bis 120° C, besonders bevorzugt bei 90 bis 110° C, durchgeführt.

Die Reaktionszeit ist u. a. abhängig von dem eingesetzten Benzalhalogenid, der Ameisensäurekonzentration und der Temperatur. Die Reaktionszeit beträgt im allgemeinen etwa 0,1 bis 20, meistens jedoch 0,2 bis 10 Stunden.

Für die Durchführung des erfindungsgemäßen Verfahrens ist es nicht erforderlich, in Gegenwart von inerten organischen Lösungs- oder Verdünnungsmitteln zu arbeiten. Gegebenenfalls können aber inerte organische Lösungs- oder Verdünnungsmittel, wie halogenierte Kohlenwasserstoffe mit 1 bis 5 Kohlenstoffatomen oder halogenierte oder alkylierte Aromaten, dem Reaktionsgemisch zugegeben werden.

Zum Beispiel werden als inerte organische Lösungs- oder Verdünnungsmittel genannt: Tetrachlorkohlenstoff, Dichlorethan, Tetrachlorethan, Hexachlorethan, Benzol, Toluol, Chlorbenzol und Dichlorbenzol, bevorzugt Toluol, Chlorbenzol.

Die Menge der inerten organischen Lösungs- oder Verdünnungsmittel, die gegebenenfalls zugesetzt wird, kann in weiten Grenzen schwanken und beträgt im allgemeinen 10 bis 100 Vol.-%, bevorzugt nicht mehr als die Menge, die erforderlich ist für die Bildung einer genügend rührbaren Suspension bei festen Einsatzprodukten und/oder Endprodukten.

Das erfindungsgemäße Verfahren kann so durchgeführt werden, daß man ein Gemisch aus Ameisensäure und Benzalhalogenid, dem gegebenenfalls ein inertes organisches Lösungs- oder Verdünnungsmittel zugesetzt wurde, unter Rühren erhitzt, bis die Entwicklung von Kohlenmonoxid und Halogenwasserstoffgas beginnt. Dies ist in der Regel bei Temperaturen von etwa 50 bis 70° C der Fall. Danach wird die Temperatur allmählich gesteigert, wobei eine merkliche Gasentwicklung zu beobachten ist. Nachdem eine Endtemperatur von etwa 105 bis 130° C im Reaktionsgemisch erreicht worden ist, wird die Reaktion bei dieser Temperatur noch so lange fortgeführt, bis die Gasentwicklung zum Stillstand gekommen ist.

Es ist auch möglich, die für die Umsetzung benötigte Ameisensäure allmählich dem vorgelegten, gegebenenfalls in einem inerten Lösungs- oder Verdünnungsmittel gelösten oder suspendierten Benzalhalogenid zuzugeben oder umgekehrt die Ameisensäure vorzulegen und das Benzalhalogenid gegebenenfalls gelöst oder suspendiert in einem inerten Lösungs- oder Verdünnungsmittel zuzugeben.

Die Aufarbeitung des Reaktionsgemisches kann in besonders einfacher Weise durchgeführt werden, da das erfindungsgemäße Verfahren sehr selektiv verläuft und außer eventuell im Überschuß vorhandener Ameisensäure und eventuell vorhandenem Wasser sowie gegebenenfalls überschüssigem inertem organischem Lösungs- oder Verdünnungsmittel keine Katalysatoren oder andere Hilfsstoffe entfernt werden müssen.

Zur Isolierung des Aldehyds genügt es, bei Einsatz von reinem Benzalhalogenid die eventuell überschüssige Ameisensäure und das eventuell zugesetzte inerte organische Lösungs- oder Verdünnungsmittel im Vakuum durch Destillation zu entfernen.

Das erfindungsgemäße Verfahren kann sowohl diskontinuierlich als auch kontinuierlich durchgeführt werden.

Im allgemeinen wird das erfindungsgemäße Verfahren bei Normaldruck durchgeführt, es ist jedoch auch möglich, das Verfahren bei Überdruck durchzuführen. Wenn das Verfahren bei Überdruck durchgeführt wird, können beträchtlich höhere Temperaturen angewendet werden. Dadurch lassen sich auch schwer hydrolisierbare Benzalhalogenide, wie 2,6-Dichlorbenzalhalogenid, in einer angemessenen Reaktionszeit umsetzen.

Nach dem erfindungsgemäßen Verfahren erhält man die aromatischen Aldehyde gewöhnlich in Ausbeuten von 95 bis 100% der Theorie und in Reinheiten von über 90%. Werden reine Benzalhalogenide eingesetzt, so kann die Reinheit über 99% betragen.

Die Vorteile des erfindungsgemäßen Verfahrens liegen in den guten Ausbeuten und in der einfachen und damit wirtschaftlichen Aufarbeitungsweise des Reaktionsgemisches, bei der eine aufwendige Abtrennung von unerwünschten Nebenprodukten sowie von Katalysatoren entfällt.

Dabei überrascht es außerordentlich, daß das erfindungsgemäße Verfahren trotz Abwesenheit von Katalysatoren gegenüber dem Stand der Technik höhere Ausbeuten an aromatischen Aldehyden liefert und die Reaktion trotzdem schnell und damit wirtschaftlich verläuft.

Die nachfolgenden Beispiele sollen das erfindungsgemäße Verfahren verdeutlichen, ohne es jedoch auf diese Beispiele einzuschränken.

## Beispiel 1

80,0 g (0,243 Mol) 4-Brombenzalbromid und 20,0 g 85%ige Ameisensäure werden unter Rühren aufgeheizt. Bei ca. 90° C beginnt eine rege Gasentwicklung, die nach 4 Stunden bei 107° C beendet ist. Im Wasserstrahlvakuum werden 4,1 g Ameisensäure und 44,5 g 4-Brombenzaldehyd abdestilliert. Der Benzaldehyd kristallisiert nach kurzem Stehen (Schmp. 57—58° C). Der Destillationsrückstand von 0,8 g besteht aus 4-Brombenzoesäure. Die Ausbeute an 4-Brombenzaldehyd beträgt 99,0% der Theorie.

## Beispiel 2

160,9 g eines Gemisches aus 1,8% 4-Chlorbenzylchlorid, 57,9% 4-Chlorbenzalchlorid und 40,2% 4-Chlorbenzotrichlorid werden mit 72,8 g 85%iger Ameisensäure unter Rühren aufgeheizt. Bei ca. 60° C beginnt die Gasentwicklung, die nach 10 Stunden bei 100° C beendet ist. Im Verlauf der Reaktion bildet sich eine Suspension, da die 4-Chlorbenzoesäure ausfällt. Bei der Destillation werden neben überschüssiger Ameisensäure 68,9 g 4-Chlorbenzaldehyd (95,7% Aldehyd, 4,3% 4-Chlorbenzylchlorid) erhalten. Der Destillationsrückstand beträgt 43,9 g (Fp.: 240 bis 241° C) und besteht aus 4-Chlorbenzoesäure (99,9%ig). Die Ausbeute an Aldehyd beträgt 98,5% der Theorie, die Ausbeute an Benzoesäure ist 99,7% der Theorie.

Beispiel 3

100,0 g eines Gemisches aus 0,2% 2-Chlorbenzylchlorid, 80,2% 2-Chlorbenzalchlorid und 19,6% 2-Chlorbenzotrichlorid werden mit 53,0 g 85%iger Ameisensäure, wie im Beispiel 2 beschrieben, umgesetzt. Nachdem die Gasentwicklung beendet ist, wird das Reaktionsgemisch über eine 10-cm-Vigreux-Kolonne destilliert. Man erhält 31,9 g Ameisensäure, 56,9 g 2-Chlorbenzaldehyd (96,9% Aldehyd, 0,4% 2-Chlorbenzylchlorid, 2,6% 2-Chlorbenzalchlorid). Der Destillationsrückstand beträgt 13,3 g (Fp.: 137 bis 138° C); und besteht aus 2-Chlorbenzoesäure (99,9%ig).

Die Ausbeute an Aldehyd beträgt 98,7% der Theorie, die Ausbeute an Benzoesäure ist 99,5% der Theorie.

Beispiel 4

76,5 g (0,25 Mol) 4-tert.-Butylbenzalbromid werden mit 11,5 g 85%iger Ameisensäure unter Rühren erhitzt (110° C). Nach 11 Stunden ist die Gasentwicklung beendet. Im Wasserstrahlvakuum werden Reste von Ameisensäure, Wasser und HBr entfernt. Der Rückstand besteht aus 40,1 g 4-tert.-Butylbenzaldehyd (98,9% Aldehyd, 0,9% 4-tert.-Butylbenzoesäure). Die Ausbeute an Aldehyd beträgt 97,9% der Theorie.

Beispiel 5

80,5 g (0,5 Mol) Benzalchlorid werden mit 16,5 g 30%iger Ameisensäure unter Rühren erhitzt (105° C). Nach 19 Stunden ist die Gasentwicklung beendet. Im Wasserstrahlvakuum werden Reste von Ameisensäure, Wasser und HCl entfernt. Der Rückstand besteht aus 52,8 g Benzaldehyd (99,9%ig). Dies entspricht einer Ausbeute von 99,4% der Theorie.

Beispiel 6

29,4 g (0,1 Mol) 2-Dibrommethylbenzoesäure werden mit 30,0 g 50%iger Ameisensäure unter Rühren erhitzt (107° C). Nach ca. 3 Stunden ist die Suspension unter Gasentwicklung in eine homogene Lösung übergegangen. Nach 7 Stunden ist die Gasentwicklung beendet.

Nachdem im Wasserstrahlvakuum Reste von Ameisensäure, Wasser und HBr entfernt sind, bleiben 14,0 g 3,3'-Diphthalidylether (Fp.: 225 bis 228° C). Dies entspricht einer Ausbeute von ca. 99% der Theorie.

**Patentansprüche**

1. Verfahren zur Herstellung von aromatischen Aldehyden der Formel

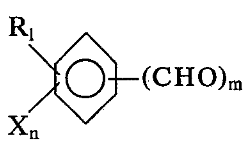

worin

R für Wasserstoff, eine Carboxylgruppe, einen Alkylrest mit 1 bis 6 Kohlenstoffatomen, einen Cycloalkylrest mit 5 bis 9 Kohlenstoffatomen, einen gegebenenfalls mit Halogen ein- oder mehrfach substituierten Phenylrest oder einen gegebenenfalls mit Halogen ein- oder mehrfach substituierten Phenoxyrest steht,
X für Halogen steht,
l die Zahlen 0, 1, 2 bedeutet,
m die Zahl 1, 2 bedeutet und
n die Zahlen 0, 1, 2, 3 bedeutet,

durch Umsetzung der entsprechenden Benzalhalogenide mit Ameisensäure bei erhöhter Temperatur, dadurch gekennzeichnet, daß man die Umsetzung mit der 0,3- bis 7fachen stöchiometrischen Menge Ameisensäure ohne Verwendung eines Katalysators durchführt.

2. Verfahren nach Anspruch 1, dadurch gekennzeichnet, daß man die Umsetzung mit der 0,5- bis 5fachen stöchiometrischen Menge Ameisensäure durchführt.

3. Verfahren nach Ansprüchen 1 und 2, dadurch gekennzeichnet, daß man die Umsetzung mit einer 30- bis 95gew.-%igen wäßrigen Ameisensäure durchführt.

4. Verfahren nach Ansprüchen 1 und 2, dadurch gekennzeichnet, daß man die Umsetzung mit einer 85gew.-%igen wäßrigen Ameisensäure durchführt.

**Claims**

1. Process for the preparation of aromatic aldehydes of the formula

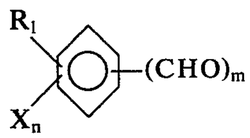

wherein

R represents hydrogen, a carboxyl group, an alkyl radical with 1 to 6 carbon atoms, a cycloalkyl radical with 5 to 9 carbon atoms, a phenyl radical optionally monosubstituted or polysubstituted by halogen, or a phenoxy radical optionally monosubstituted or polysubstituted by halogen,
X represents halogen,
l denotes the number 0, 1 or 2,
m denotes the number 1 or 2 and
n denotes the number 0, 1, 2 or 3,

by reacting the corresponding benzal halides with formic acid at elevated temperature, characterised in that the reaction is carried out with 0.3 to 7 times the stoichiometric amount of formic acid without using a catalyst.

2. Process according to Claim 1, characterised in that the reaction is carried out with 0.5 to 5 times the stoichiometric amount of formic acid.

3. Process according to Claims 1 and 2, characterised in that the reaction is carried out with aqueous formic acid of 30 to 95% strength by weight.

4. Process according to Claim 1 and 2, characterised in that the reaction is carried out with aqueous formic acid of 85% strength by weight.

**Revendications**

1. Procédé de préparation d'aldéhydes aromatiques de formule

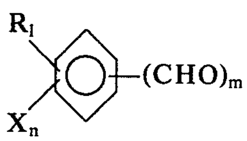

dans laquelle

R représente l'hydrogène, un groupe carboxyle, un groupe alkyle en $C_1$—$C_6$, un groupe cycloalkyle en $C_5$—$C_9$, un groupe phényle éventuellement mono- ou poly-substitué par des halogènes ou un groupe phénoxy éventuellement mono- ou poly-substitué par des halogènes,
X représente un halogène,
l est égal à 0, 1 ou 2,
m est égal à 1 ou 2, et
n est égal à 0, 1, 2 ou 3,

par réaction des halogénures de benzylidène correspondants avec l'acide formique à température élevée, caractérisé en ce que l'on effectue la réaction avec une quantité d'acide formique représentant de 0,3 à 7 fois la quantité stoechiométrique sans utiliser de catalyseur.

2. Procédé selon la revendication 1, caractérisé en ce que l'on effecture la réaction avec une quantité d'acide formique représentant de 0,5 à 5 fois la quantité stoechiométrique.

3. Procédé selon les revendications 1 et 2, caractérisé en ce que l'on effectue la réaction avec un acide formique aqueux à une concentration de 30 à 95% en poids.

4. Procédé selon les revendications 1 et 2, caractérisé en ce que l'on effecture la réaction avec un acide formique aqueux à la concentration de 85% en poids.